# EUROPEAN PATENT SPECIFICATION

(11) **EP 1 015 025 B1**
(45) Date of publication and mention of the grant of the patent: **15.12.2004**
(21) Application number: 98949378.8
(22) Date of filing: 18.09.1998
(51) Int. Cl.: A61K 39/00, A61K 35/14

(54) **T-CELL VACCINATION FOR THE TREATMENT OF MULTIPLE SCLEROSIS**
T-ZELLIMPFUNG ZUR BEHANDLUNG VON MULTIPLE SCLEROSIS
VACCINATION A L'AIDE DE LYMPHOCYTES T POUR LE TRAITEMENT DE LA SCLEROSE EN PLAQUES

(30) Priority: 19.09.1997 US 59534 P; 17.09.1998 US 156509
(43) Date of publication of application: 05.07.2000
(73) Proprietor: UNIVERSITY OF SOUTHERN CALIFORNIA, Los Angeles, CA 90007-4344 (US)
(72) Inventor: WEINER, Leslie, P., Los Angeles, CA 90005 (US); CORREALE, Jorge, D., Buenos Aires (AR)
(74) Representative: Fiener, Josef
(86) International application number: PCT/US1998/019572
(87) International publication number: WO 1999/013904

(56) References cited:
- WO-A-90/10449
- WO-A-91/15225
- J. ZHANG ET AL.: "MHC-restricted depletion of human myelin basic protein-reactive T cells by T cell vaccination." SCIENCE, vol. 261, no. 5127, 10 September 1993, pages 1451-1454, XP002091570 Washington, DC, USA cited in the application
- J. ZHANG ET AL.: "Myelin basic protein-reactive T cells in multiple sclerosis: Pathologic relevance and therapeutic targeting." CYTOTECHNOLOGY, vol. 16, no. 3, 1994, pages 181-187, XP002091571 Dordrecht, The Netherlands
- P. STINISSEN ET AL.: "Vaccination with autoreactive T cell clones in multiple sclerosis: Overview of immunological and clinical data." JOURNAL OF NEUROSCIENCE RESEARCH, vol. 45, no. 4, 15 August 1996, pages 500-511, XP002091572 New York, NY, USA
- R. MEDAER ET AL.: "Prospects for T cell vaccination in multiple sclerosis." BIODRUGS, vol. 8, no. 1, July 1997, pages 1-5, XP002091573 Auckland, New Zealand
- J. CORREALE ET AL.: "Isolation and characterization of CD8+ T-cell clones (TCCs) that recognize autologous-activated CD4+ TCCs from chronic progressive multiple sclerosis (MS) patients: Implications for immunoregulation in MS." NEUROLOGY, vol. 46, no. 2 suppl. S, February 1996, page A280 XP002091574 Minneapolis, MN, USA
- L. WEINER ET AL.: "Isolation and characterization of CD8+ and gamma-delta T cells specific for autologous autoreactive CD4+ T cell clones from multiple sclerosis patients and healthy control subjects." NEUROLOGY, vol. 48, no. 3 suppl. 2, August 1997, pages A59-A60, XP002091575 Minneapolis, MN, USA
- J. VAN LAAR ET AL.: "Effects of inoculation with attenuated autologous T cells in patients with rheumatoid arthritis." JOURNAL OF AUTOIMMUNITY, vol. 6, no. 2, April 1993, pages 159-167, XP002091576 London, GB cited in the application
- L. MORELAND ET AL.: "Vaccination against rheumatoid arthritis." BIODRUGS, vol. 8, no. 2, August 1997, pages 87-95, XP002091577 Auckland, New Zealand

## Description

### BACKGROUND OF THE INVENTION

### Field of the Invention

The present invention relates generally to the field of immunotherapy and to treatments for autoimmune diseases. In particular, the invention relates to methods of using T-cells as vaccines for treating autoimmune diseases, including multiple sclerosis.

### Description of the Related Art

Autoimmune diseases affect 5-7% of the adult population in Europe and North America. (Sinha AA, MT Lopez, *et al*. (1990) *Science* **248**:1380-1387). This group of diseases has a major socioeconomic impact, not only because they are accompanied by long life expectancies, but also because they strike individuals in their most productive years. For example, the patients who get multiple sclerosis (MS) are predominantly women between the ages of 18 and 40.

Autoimmune diseases are thought to result from an uncontrolled immune response directed against self antigens. In contrast, individuals who do not mount an autoimmune response to self antigens are thought to have control over these responses and are believed to by "tolerant" of self antigens. Although the etiology of MS remains unknown, several lines of evidence support the hypothesis that autoimmunity plays a significant role in the development of the disease (Martin R, HF McFarland, *et al*. (1992) *Annu. Rev. Immunol.* **10**:153-187). In MS, there is evidence that the uncontrolled immune response is against the white matter of the central nervous system and more particularly to myelin proteins that are located in the white matter. Ultimately, the myelin sheath surrounding the axons is destroyed. This can result in paralysis, sensory deficits and visual problems. MS is characterized by a T-cell and macrophage infiltrate in the brain. Presently, the myelin proteins thought to be the target of an immune response in MS include myelin basic protein (MBP), proteolipid protein (PLP), myelin associated glycoprotein (MAG), and myelin-oligodendrocyte glycoprotein (MOG). Also there is an increasing body of evidence that the T-cell receptor has extraordinary flexibility, allowing it to react to many different proteins (Brock R, KH Wiesmuller, *et al*. (1996) *Proc. Natl*. *Acad*. *Sci. (USA)* **93**:13108-13113; Loftus DJ, Y Chen, *et al.* (1997) *J*. *Immunol.* **158**:3651-3658).

Further support for this concept is based on studies of experimental allergic encephalomyelitis (EAE), an animal model with clinical and pathologic similarities to MS. (Alvord, *et al*. Experimental allergic encephalomyelitis. A useful model for multiple sclerosis. New York: Alan R. Liss, 1984) In both EAE and MS, myelin basic protein (MBP), proteolipid protein (PLP), and MOG are thought to be the main target antigens for autoreactive T-cells (Brostoff SW and DW Mason (1984) *J. Immunol* **133**:1938-1942; Tabira and Kira, 1992). Myelin associated glycoprotein (MAG) may be important in MS but does not produce EAE in experimental models.

The autoimmune nature of MS has to be explained in relation to the epidemiology that supports a role for an environmental agent. This is presumably a virus or viruses, or other microbes. The natural history of the disease also suggests that infection may trigger exacerbations in certain patients. The debate over a role for persistent infection versus recurrent infection as the instigator of autoimmune disease remains unsettled. The mechanism of virus interaction may be molecular mimicry of host protein by invading microorganisms.

Immunologic self-tolerance appears to be achieved primarily by clonal deletion of autoreactive T-cells in the thymus during negative selection, and in peripheral lymphoid tissue post maturation. However, even in healthy individuals, not all autoreactive T-cells are deleted in the thymus. Autoreactive T-cells represent part of the normal T-cell repertoire and can be isolated from normal individuals without autoimmune diseases (Correale J, M McMillan, *et al*. (1995) *Neurology* **45**:1370-1378). Thus, autoreactive T-cells may exist in the periphery without causing disease. This suggests that post-thymic mechanisms control autoreactive T-cells to provide protection from immunological attacks against self. A number of mechanisms are operative *in vivo* to regulate autoreactive T-cells. Such mechanisms may involve antigen-directed T-cell clonal anergy or regulatory cellular networks that influence autoreactive T-cells by interacting with their idiotypes or structures of their state of activation ergotype (Lohse AW, F Mor, *et al*. (1989) *Science* **244**:820-822; Ben-Nun A, H Wekerle, and IR Cohen (1981) *Nature* **292**:60-61; Holoshitz J, Y Naparstek, *et al*. (1983) *Science* **219**:56-58; and Maron R, R Zerubavel, *et al*. (1983) *J Immunol*. **131**:2316-2322). The mechanisms regarding the signaling molecules on target T-cells that elicit the idiotypic interactions are still not understood, but are thought to involve both CDR2 and CDR3 hypervariable regions of the T-cell receptor Vp chain (Saruhan-Direskendi G, F Weber, *et al*. (1993) *Eur*. *J*. *Immunol*. **23**:530-536). In patients with MS, resistance of T-cells to a variety of regulatory controls may account for the entry of autoimmune diseases into a chronic progressive phase (Correale J, W Gilmore, *et al* (1996) *Nature Medicine* **2**:1354-1360). Several factors make treatment of MS particularly difficult. For example, the patient's aberrant immune response to new myelin antigens expands during the period the patient appears to be in remission (Correale J, M McMillan, *et al*. (1995) *Neurology* **45**:1370-1378). In addition, in chronic MS, in contrast to acute disease, the T-lymphocytes are able to present antigen to themselves without a true antigen-presenting cell, thus further amplifying the abnormal response to myelin proteins (Correale J, W Gilmore, *et al*. (1995) *J*. *Immunol*. **154**:2959-2968).

The course of MS is highly variable. Most typically, the disease is characterized by a relapsing pattern of acute exacerbations followed by periods of stability (remissions). However, in many cases this pattern evolves after some years into a secondary progressive course, in which the clinical condition slowly deteriorates. Moreover, in some patients the disease is relentlessly progressive from the onset (primary progressive MS).

The goal of immunologic therapy is to restore tolerance without suppressing the entire immune system and causing complications such as opportunistic infection, hemorrhage, and cancer. A variety of therapeutic approaches are now available in humans. These include general cytotoxic agents (cytoxan) that lack selectivity. Other examples include cyclosporin, and FK 506, that work on the cytokine IL-2 and its receptor; radiation, which induces apoptosis and cell death; corticosteroids; blockade of the MHC that prevents antigen binding; blockade of the invariant TCR-CD3 complex; blockade of proinflammatory cytokines and their receptors such as IL-2 and gamma interferon and anti inflammatory cytokines such as beta interferon; anti adhesion molecules such as CD2 of LFA; anti T-cell activation by antibody to CD4; and use of anti inflammatory cytokines such as IL-10, TGF-β and IL-4. All of these treatments are antigen non-specific and therefore cannot differentiate physiologic from pathologic responses.

Suppression of the immune system in a more specific way is more desirable for control of the response to self antigens without down-regulating the entire immune system. Several specific immunotherapies have been hypothesized and tested in recent years, many of which are impractical or do not work in humans. For example, high affinity peptides can be synthesized which interact with MHC class II molecules and prevent the binding of encephalitogenic peptides, thereby preventing the activation of pathogenic T-cells (A Franco *et al*. (1994) The Immunoloigist **2**:97-102). This approach is disadvantageous in that it is difficult to obtain effective concentrations of inhibitor peptides *in vivo* (Ishioka GY, L Adorini, *et al*. (1994) *J*. *Immunol*. **152**:4310-4319). In an alternate strategy, peptides which are analogs of encephalitogenic sequences (altered peptide ligands) have been shown to antagonize the T-cell receptors of antigen-specific T-cells, rendering them unreactive, although the exact mechanism is at present unknown (Jameson SC, FR Carbone, *et al*. (1993) *J. Exp. Med.* **177**:1541-1550; Karin N, DJ Mitchell, *et al*. (1994) *J*. *Exp*. *Med*. **180**:2227-2237; and Kuchroo VK, JM Greer, *et al*. (1994) *J*. *Immunol*. **153**:3326-3336). Oral administration of myelin has been tested and found in EAE to induce a state of immunological unresponsiveness thought to be mediated by the induction of a suppresser T-cell or of anergy (Weiner HL, A Friedman, *et al*. (1994) *Annu. Rev Immunol*. **12**:809-837; Whitacre CC, IE Gienapp, *et al*. (1991) *J*. *Immunol.* **147**:2155-2163; and Khoury SJ, WW Hancock, *et al*. (1992) *J*. *Exp*. *Med.* **176**:1355-1364). This treatment has been found to be efficacious for some but not all individuals (Weiner HL, GA Mackin, *et al*. (1993) *Science* **259**:1321-1324). A most recent large phase II/III trial has not shown efficacy in remitting/relapsing MS (unpublished results).

Some studies have focused on the antigen or the T-cell that is producing the damage. For example, studies have shown that pathogenic T-cells capable of inducing autoimmune diseases in animal models can be rendered "avirulent" by attenuation and can be administered as vaccines to prevent subsequent indication of the disease (Cohen IR (1989) *Cold Spring Harbor Symposia on Quantitative Biology* **54**:879-884). In these studies, T-cell vaccination induced effective anti-idiotypic and anti-ergotypic T responses. Recent studies have shown that T-cell vaccination with the avirulent cells in primates and humans afflicted with rheumatoid arthritis and MS is technically feasible and non-toxic. It also has been shown that it is possible to target and deplete a population of autoreactive T-cells involved in the autoimmune process using T-cell vaccination. Results, however, were not definitive (Hafler DA, Cohen IR, *et al*. (1992) *Clin*. *Immunol*. *and Immunopathol* **62**:307-313; Lohse AW, NPM Bukker, *et al*. (1993) *J*. *Autoimmunity* **6**:121-130; van Laar JM, AMM Miltenburg, *et al*. (I993) *J*. *Autoimmunity* **6**:159-167; and Zhang J, R Medaer, *et al*. (1993) *Science* **261**:1451-1454). However, these experimental treatments for MS have targeted only myelin basic protein activated T-cells. It is highly probable that MBP-reactive T-cells represent only a small group of the autoreactive T-cells responsible for the progression of the disease.

Further report of the T-cell vaccination of multiple sclerosis has been published (Stinissen P. et al., 1996, *J. Neurosci Res.* **45**: 500-511; Medaer R. et al., 1997, *Biodrugs* **8**: 1-5). Stinissen et al. report on a pilot trial of T-cell vaccination in a limited group of MS-patients. Thereby, autologous attenuated myelin basic protein reactive T-cell clones have been injected. The cells are prepared by culturing autologous peripheral mononuclear cells in the presence of myelin basic protein and antigen presenting cells.

There currently is no effective therapy for primary or secondary MS. Thus it is evident that improvements are needed to treat MS and other autoimmune disorders with an non-toxic, effective, immunospecific approach.

### THE INVENTION

### Summary of the Invention

The present invention addresses the disadvantages present in the prior art. One aspect of the invention is a vaccine for the treatment of MS. The vaccine is comprised of attenuated T-cells that target more than one myelin protein. In a preferred embodiment of the invention, the T-cells in the vaccine are autologous. In another preferred embodiment of the invention, the T-cells target more one myelin protein. Another aspect of the invention is the use of said attenuated T-cells for the manufacture of a medicament for medicating an immune response in a human MS patient. Yet another aspect of the invention is a method of making a vaccine comprised of attenuated T-cells for the treatment of MS. In another preferred embodiment of the invention, the T-cells are cultured in the presence of a mixture of bovine myelin proteins.

### Brief Description of the Drawings

Figure 1A-1D (collectively referred to herein as Figure 1) shows EDSS scores and changes in the frequencies of circulating bovine myelin-reactive T-cells.
Figure 2A-2C (collectively referred to herein as Figure 2) shows the number of interferon-gamma and interleukin-2 secreting T-cells reactive to bovine myelin proteins.
Figure 3A-3D (collectively referred to herein as Figure 3) illustrates changes in the frequencies of T-cells reactive to MBP, PLP and MOG peptides.
Figure 4 demonstrates inhibition of the proliferation of inoculates by anti-myelin reactive T-cell lines.
Figure 5 illustrates cytotoxicity of the anti-myelin reactive T-cells.
Figure 6 shows MHC restriction of anti-myelin reactive T-cells.

### Detailed Description of the Invention

### Definitions

Anti-ergotypic means against structures of a state of activation.

Anti-idiotypic means against the characteristics of an autoreactive T-cell.

Autoreactive means a B or T-cell that reacts against the host's own tissues.

As stated above, the present invention relates to a vaccine for the treatment of MS, methods of producing the vaccine, and methods for its use. The vaccine is comprised of attenuated T-cells that are presumed to be autoreactive. Preferably, the T-cells are obtained from the patient to be vaccinated. A further clarification of the target T-cell sequences (including sequences for T-cell receptors) recognized by anti-idiotypic and anti-ergotypic T-cells may be used to design synthetic peptides corresponding to predominant sequences characteristic of pathogenic myelin reactive T-cells. Therefore, this approach may be used to eliminate the need for autologous T-cell vaccination in which each patient needs his or her own vaccine. Preferably, T-cells are removed from the patient by leukapheresis. Pathogenic T-cells are estimated to occur at a frequency of between 1:20,000 to 1:40,000 peripheral blood mononuclear cells (PBMCs). Therefore, to effectively sample the repertoire it is necessary to obtain as many cells as possible. Leukapheresis provides on the order of 1 x 10⁹ T-cells. A sufficient number of autologous PBMCs must also be obtained to use as feeder cells during the growing of autoreactive T-cells for vaccine development.

Preferably, the PBMCs obtained are cultured in presence of cow myelin proteins or synthetic complete human myelin proteins as they are identified and become available. The cells that respond to myelin proteins are selected and expanded This is accomplished by culturing the cells in the presence of specific myelin antigens. The non-specific cells are lost in the process.

The cells are attenuated. Preferably, this is performed by irradiating the cells at 12,000 Rads. Since these T-cells have been selected for their reactivity to myelin, they must be killed or they will attack the patient's myelin when injected. The irradiated cells are not frozen, although the fresh cells can then be stored frozen and then irradiated and used for future injection into the patient.

Patients preferably receive subcutaneous injections of attenuated T-cells every 4-6 weeks. The number of cells is preferably 40,000,000. However, the optimum number of cells may vary by patient. The preferred range of cells/vaccination is between 30 and 80 x 10⁶. Previous T-cell vaccination protocols in multiple sclerosis and rheumatoid arthritis have used 30-60 x 10⁶ cells/vaccination without serious side effects (van Laar JM, AMM Miltenburg, *et al*. (1993) *J*. *Autoimmunity* **6**:159-167; Zhang J, R Medaer, *et al*. (1993) *Science* **261**:1451- 1454). Inoculations are given in 4-6 week intervals for 6 months and depending on clinical, immunologic and MRI data, the dose and interval for the injections may be adjusted.

Preferably, if after the first 2 inoculations, patients do not respond clinically to the number of myelin autoreactive T-cells administered, a dose-escalation administration is started. The number of inoculated cells may be increased 25% each 3 months to the point at which adverse reactions appeared. This type of gradual escalation can provide information on the upper limits of safety and indicate a dose range in which efficacy studies could be conducted. If no escalation is necessary, injections are given in 3 month intervals for the next 18 months.

Adverse reactions may be reactions such as: 1) systemic symptoms that require in-patient hospitalization; 2) a phase of increasing disability that progresses two or more steps in EDSS scale over two consecutive scheduled neurologic evaluations; 3) CD4+ lymphocyte counts below 500 cells/mm³.

Without wishing to be bound by any particular theory, the mechanism of action for the vaccine is believed to be a host response to the T-cell receptor(TCR) variable region on the irradiated pathogenic T-cell that comprises the vaccine. This region is the only area thought to be different on the pathogenic T-cell as compared to other naive or activated T-cells. The approach described herein is based on the hypothesis that there are many V_{α} and V_{β} families involved since progressive MS has so many different antigen specific responses and immunodominant epitopes may differ from patient to patient. This allows T-cells from each patient to be activated against epitopes it has seen *in vivo.* when inactivated by radiation, the TCRs become antigens and induce either an anti-idiotypic antibody or a T-cell response against the V_{α} and V_{β} regions of many different pathogenic T-cells in that patient. The result is either down regulation or killing of existing and future pathogenic responses. Since it is a "killed" vaccine, it may be necessary to give a booster once a year to perpetuate the anti-myelin specific T-cells inactivation or killing.

### Example 1

Four patients with definite secondary progressive MS and without response to any other available treatment were studied. Age range was 32-45 years, and no sex criteria was used. Progression of at least one unit in the Kurtzke scale occurred in the year prior to entry. The patients were otherwise healthy and had no other diseases to explain their neurologic conditions. All patients were free of immunotherapy for 60 days, or steroids for at least 90 days prior to the start of this protocol. Two patients had never received any prior treatment for MS.

Peripheral blood mononuclear cells (PBMCs) were obtained by leukapheresis. Approximately 10⁵ - 10⁶ myelin protein specific T-cells can be obtained per apheresis. To obtain 40 x 10⁶ cells for vaccination required a 40-400 fold expansion. Leukapheresis was performed prior to vaccination. There were 6-8 weeks between the first apheresis and the first injection.

Routine blood samples were obtained for immunologic safety by standard venipuncture. Patients were asked to donate 50-70 cc of blood at monthly intervals for three months and then at two-months intervals for 21 months for routine assessment for safety measures and to assess the effect of the vaccination program on the immune system.

To establish autoreactive T-cell lines PBMCs were cultured in serum-free media supplemented with gentamicin and stimulated with bovine total myelin proteins prepared according to standard protocols (Correale J, M McMillan, *et al*. (1995) *Neurology* **45**:1370- 1378) and sterilized by filtration through a 0.22 micron filter. After 5-7 days cells were expanded using 50 U/ml of recombinant human IL-2 (Cetus). T-cell lines were re-stimulated after 10-14 days using autologous irradiated PBMCs as antigen presenting cells (APCs) and bovine myelin proteins. Cycles of restimulation and expansion were repeated weekly until the response to myelin antigens detected in proliferation assays exceeded the response to control antigens by three fold. At that time, usually following 3-4 cycles of restimulation and expansion, activated myelin specific T-cells were separated from APCs using Ficoll gradient separation, washed in sterile phosphate buffered saline (PBS) and irradiated for attenuation (12000 rads Cs¹³⁷). Aliquots of living cells were frozen (prior to irradiation) for future injections administered every 6-12 weeks. For these injections, cells were thawed and then irradiated just prior to inoculation.

Each patient received 40 x 10⁶ cells resuspended in 1 ml of sterile PBS and injected subcutaneously (0.5 ml/arm). Prior to injection, an aliquot of the T-cell preparation was tested for bacterial growth, endotoxins, fungus, cytomegalovirus, herpes simplex, adenovirus, varicella zoster and mycoplasms (GMP). In addition, a skin test was performed using intradermal injection of 25,000-50,000 T-cells suspended in 0.1 ml of sterile PBS to test for immediate type hypersensitivity. These procedures were repeated prior to each inoculation. The patients were kept as in-patients for the first 48 hrs. following vaccination. Vaccination was repeated at 3-month intervals for the first two patients and at 6-week intervals for the second two patients for 6 months, and then all 4 patients were vaccinated at 3-month intervals.

Patients were monitored to determine whether there was any progression or improvement of neurologic deficits and neuropsychological profile. Neurological progression was defined as an increase of one or more EDSS steps maintained for more than 90 days. All patients had a baseline and annual MRI study (brain or spinal cord, according to localization of the lesions) at month 12 and month 24 after vaccination. A reduction in lesions area and in percentage change in lesions area from baseline to one year and two years for individual subjects were used as parameters for efficacy. Patients should have MRIs done at 3-month intervals to check efficacy and determine dose and frequency of the vaccine. Frequency of circulating myelin-reactive T-cells before and after each inoculation was measured to determine whether a decline in such cells correlated reciprocally with the proliferative responses of peripheral blood mononuclear cells to the inoculates

Treatment discontinuation criteria were pregnancy, CD4+ lymphocyte counts below 500 cells/mm³, occurrence of grade III or IV toxicity, use of other investigational or experimental therapies of MS, a phase of increasing disability that progresses two or more steps in the EDSS scale unremittingly over a six month period, or serious intercurrent illness precluding continued treatment with T-cell vaccine.

### Measurement of Immunological Response

T-cell response to the inoculates was examined in PBMC by using a standard 60 hr. proliferation assay, and the responses were compared with T-cell blasts prepared concurrently by PHA stimulation and resting autoreactive myelin T-cells (8-10 days after the last stimulation with antigen presenting cells and myelin). Frequency of T-cells capable of suppressing the proliferation of inoculates was measured using frequency analysis. Cultures exerting more than 60% inhibition on the proliferation of inoculates were considered as responding T-cell lines. Anti-idiotypic and anti-ergotypic responses were evaluated using standard Cr⁵¹ release assays. Patterns of cytokine secretion of anti-idiotypic, anti-ergotypic and myelin reactive T-cells were evaluated by ELISAs and ELISPOTSs. Phenotyping of the regulatory populations and fresh PBMC was studied using flow cytometry analysis.

### Results

We have recently isolated anti-idiotypic and anti-ergotypic T-cells by *in vitro* stimulation of T-cells with autologous irradiated autoreactive PLP CD4+ T-cells. These anti-idiotypic and anti-ergotypic T-cell clones express a CD8+ phenotype and lyse *in vitro* auto-reactive CD4+ PLP T-cell clones.

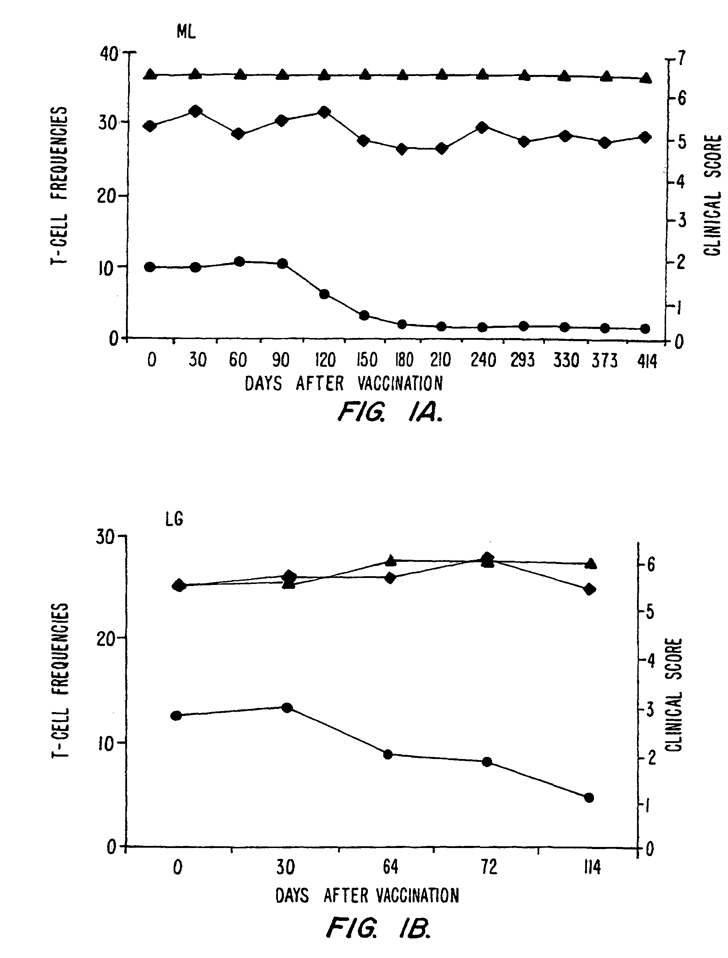

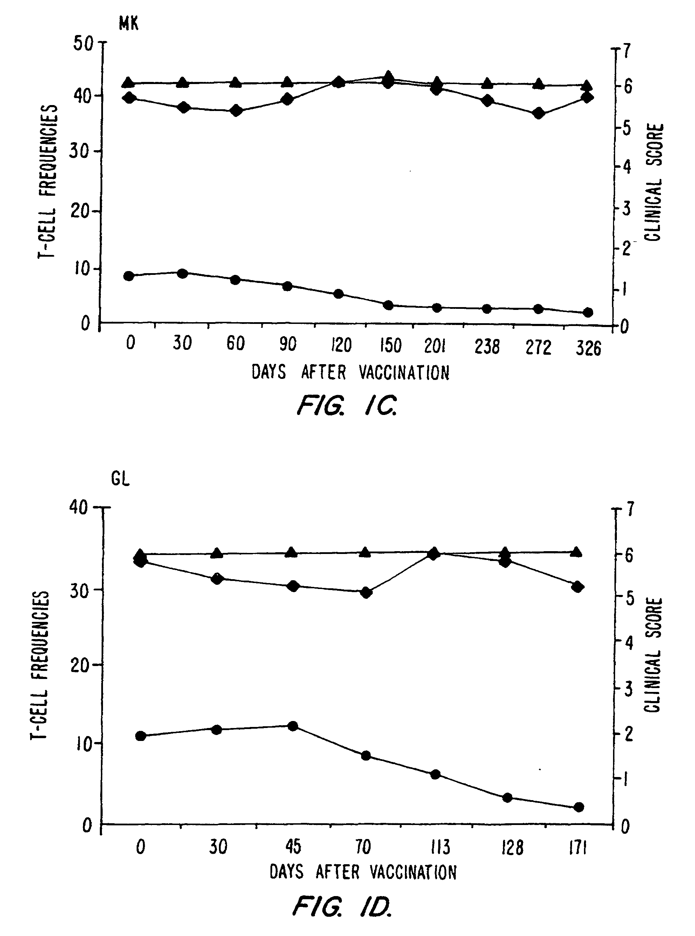

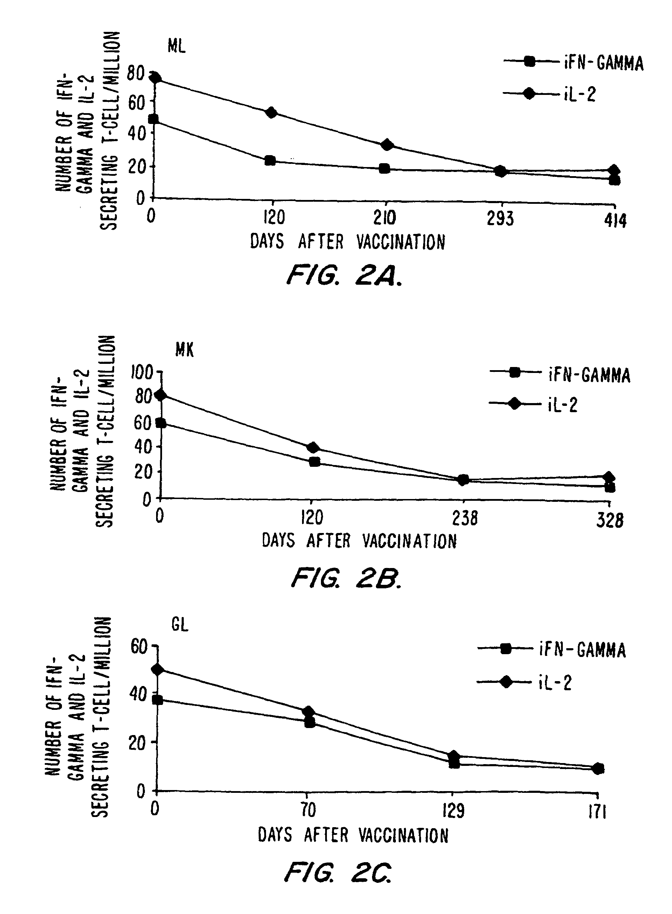

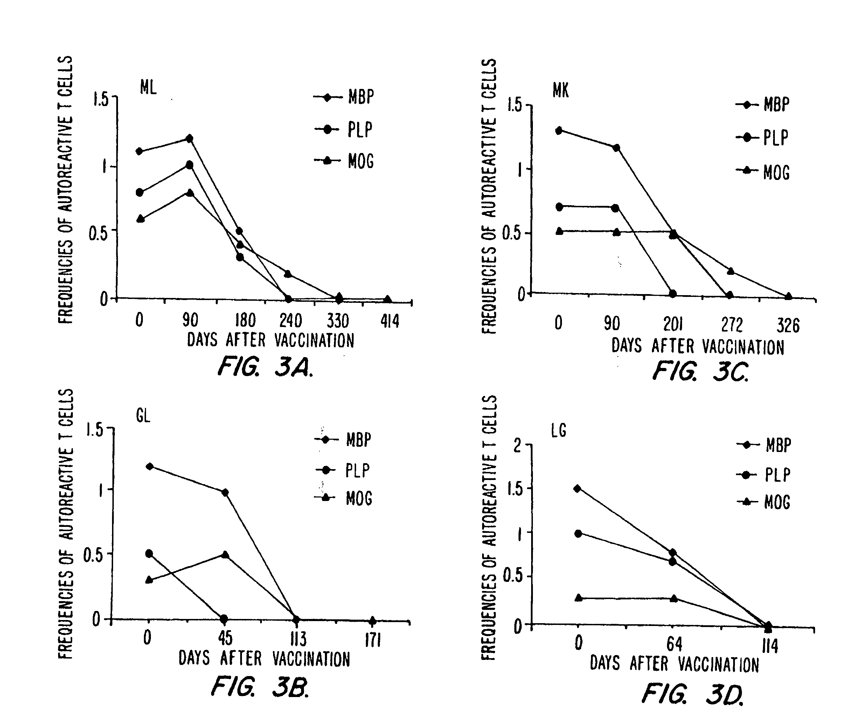

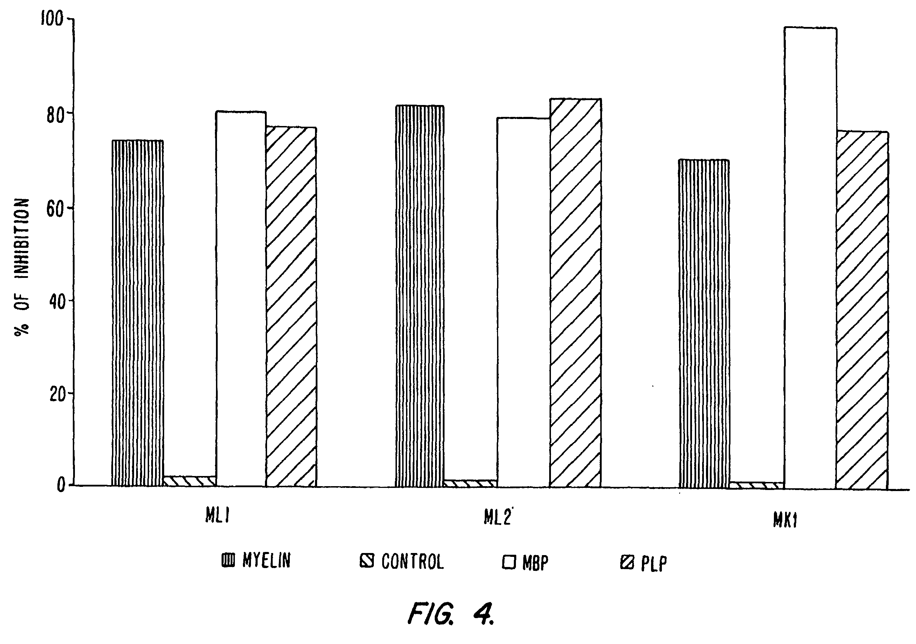

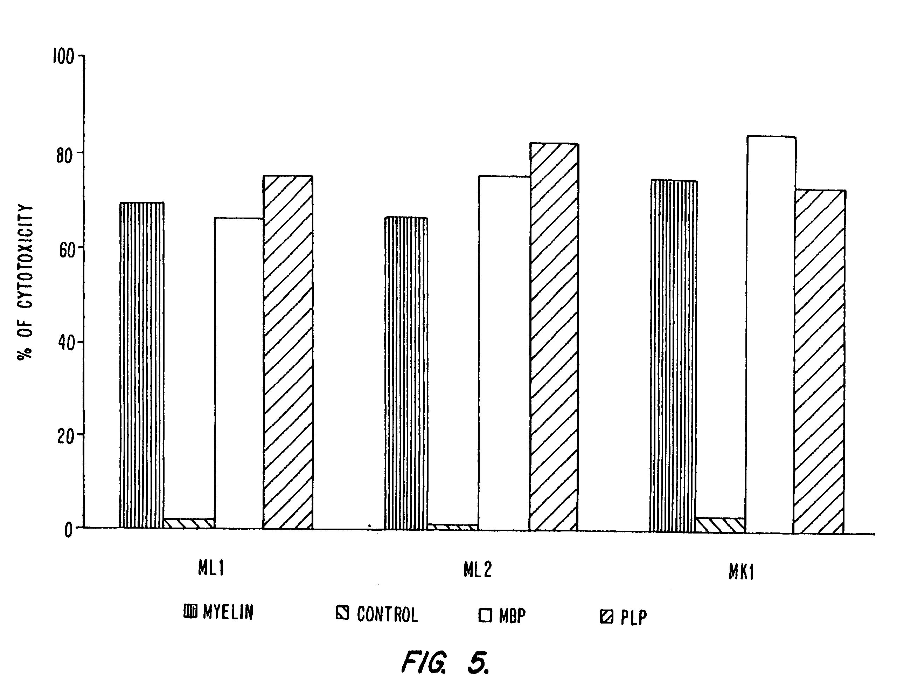

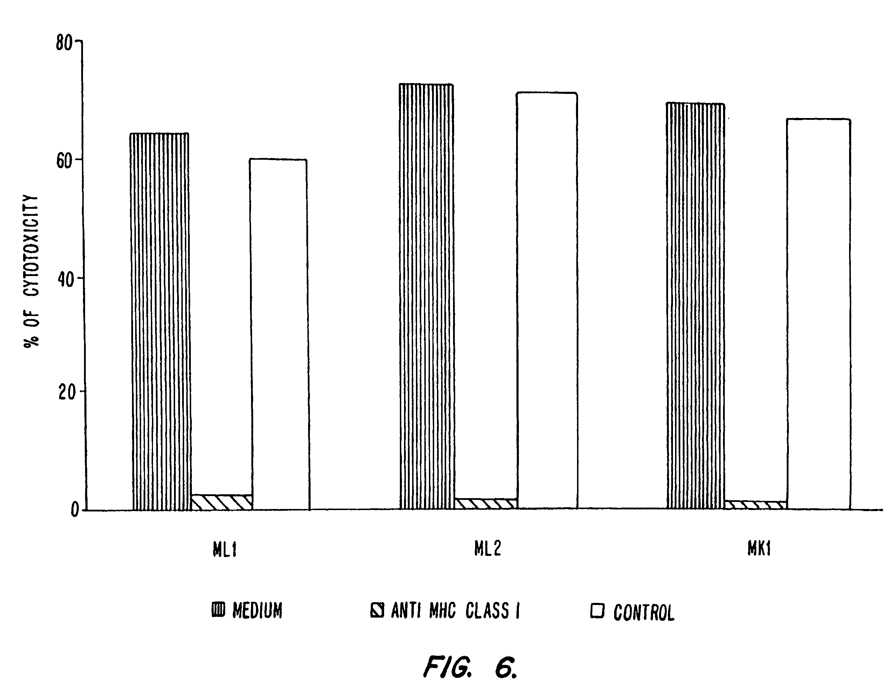

Following vaccination, three of the patients tested had no change in EDSS score in follow up testing conducted for 414 days (patient ML), 326 days (patient MK), or 171 days (patient GL). In addition, the patients had no T-cell response to bovine myelin (Figure 1). In these three patients, we observed a decrease in the number of interferon-gamma (IFN-gamma) and interleukin-2 (IL-2) secreting T-cells reactive to bovine myelin proteins (Figure 2). In all four patients, dramatic decreases in the frequencies of T-cells reactive to MBP, PLP and MOG peptides also was observed (Figure 3). Figure 4 demonstrates inhibition of the proliferation of the inoculates by anti-myelin reactive T-cell lines. Figure 5 shows the cytotoxicity of the anti-myelin reactive T-cells. Figure 6 shows MHC restriction of anti-myelin reactive T-cells.

In conclusion, our previous data support the notion that T-cells from chronic progressive MS patients are resistant to a variety of immunosuppressive mechanisms, as well as Immunomodulatory drugs phase (Correale J., W. Gilmore, *et al*. (1996) *Nature Medicine* **2**:1354-1360). These findings represent the rationale to develop new alternatives for the treatment ofprogressive MS. The broad-based immune response in this group of patients requires the widest range of antigen-specific T-cells to be inactivated. This therapeutic approach is a unique T-cell vaccine and is described in the protocol.

## Claims

1. A vaccine comprising, in an amount effective to suppress multiple sclerosis upon administration to a human, attenuated T-cells that target more than one myelin protein.

2. The vaccine of claim 1, comprising T-cells cultured in the presence of bovine total myelin proteins.

3. The vaccine of claim 2, wherein the vaccine is prepared by selecting and expanding T-cells that respond to myelin proteins.

4. The vaccine of claim 1, wherein the T-cells are derived from autologous peripheral mononuclear cells.

5. The vaccine of claim 1, wherein the T-cells are attenuated by irradiation.

6. The vaccine of claim 4, wherein the T-cells are frozen before attenuation.

7. Use of attenuated T-cells that target more than one myelin protein for the manufacture of a medicament for mediating an immune response in a human MS patient.

8. The use of claim 7, wherein the T-cells are derived from autologous peripheral mononuclear cells.

9. The use of claim 7, wherein the T-cells comprise T-cells cultured in the presence of bovine total myelin proteins.

10. The use of claim 9, wherein the T-cells are prepared by selecting and expanding T-cells that respond to myelin proteins.

11. The use of claim 7, wherein the attenuated T-cells are attenuated by irradiation.

12. The use of claim 7, wherein the T-cells are administered subcutaneously.

13. The use of claim 7, wherein the T-cells are administered in 4 to 6 week intervals.

14. The use of claim 7, wherein the T-cells are administered for approximately 18 months.

15. The use of claim 7, wherein the T-cells are administered in a first dosage of 30 x 10⁶ to 80 x 10⁶ attenuated T-cells.

16. The use of claim 15, further comprising more than one administered dosage, wherein later dosages are increased if there is no clinical response to the first dosage, up to the point of adverse reactions or of clinical response.

17. A vaccine comprising, in an amount effective to suppress multiple sclerosis, upon administration to a human, attenuated T-cells, wherein the attenuated T cells are prepared by;
culturing autologous peripheral mononuclear cells in the presence of bovine total myelin proteins;
selecting and expanding T-cells that respond to the myelin proteins; and
attenuating the T-cells by irradiation.

## Patentansprüche

1. Impfstoff, der bei Anwendung am Menschen in einer zur Unterdrückung von multipler Sklerose wirksamen Menge abgeschwächte T-Zellen umfasst, die auf mehr als ein Myelinprotein gerichtet sind.

2. Impfstoff nach Anspruch 1, der in Anwesenheit von bovinen Gesamtmyelinproteinen kultivierte T-Zellen umfasst.

3. Impfstoff nach Anspruch 2, wobei der Impfstoff durch Auswählen und Expandieren von T-Zellen hergestellt ist, die auf Myelinproteine ansprechen.

4. Impfstoff nach Anspruch 1, wobei die T-Zellen aus autogenen peripheren mononukleären Zellen hergeleitet sind.

5. Impfstoff nach Anspruch 1, wobei die T-Zellen durch Bestrahlung abgeschwächt sind.

6. Impfstoff nach Anspruch 4, wobei die T-Zellen vor der Abschwächung gefroren sind.

7. Verwendung von abgeschwächten T-Zellen, die auf mehr als ein Myelinprotein gerichtet sind, zur Herstellung eines Medikamentes zur Vermittlung einer Immunantwort in einem menschlichen MS-Patienten.

8. Verwendung nach Anspruch 7, wobei die T-Zellen aus autogenen peripheren mononukleären Zellen hergeleitet werden.

9. Verwendung nach Anspruch 7, wobei die T-Zellen in Anwesenheit boviner Gesamtmyelinproteine kultivierte T-Zellen umfassen.

10. Verwendung nach Anspruch 9, wobei die T-Zellen durch Auswählen und Expandieren von T-Zellen hergestellt werden, die auf Myelinproteine ansprechen.

11. Verwendung nach Anspruch 7, wobei die abgeschwächten T-Zellen durch Bestrahlung abgeschwächt werden.

12. Verwendung nach Anspruch 7, wobei die T-Zellen subkutan verabreicht werden.

13. Verwendung nach Anspruch 7, wobei die T-Zellen im Abstand von vier bis sechs Wochen verabreicht werden.

14. Verwendung nach Anspruch 7, wobei die T-Zellen über ca. 18 Monate verabreicht werden.

15. Verwendung nach Anspruch 7, wobei die T-Zellen in einer Erstdosierung von 30 x 10⁶ bis 80 x 10⁶ abgeschwächten T-Zellen verabreicht werden.

16. Verwendung nach Anspruch 15, die ferner mehr als eine verabreichte Dosierung umfasst, falls eine klinische Antwort auf die Erstdosierung ausbleibt, wobei spätere Dosierungen bis zum Zeitpunkt von Gegenreaktionen oder klinischer Antwort erhöht werden.

17. Impfstoff, der bei Anwendung am Menschen in einer zur Unterdrückung von multipler Sklerose wirksamen Menge abgeschwächte T-Zellen umfasst, wobei die abgeschwächten T-Zellen hergestellt sind durch:
- Kultivieren autogener peripherer mononukleärer Zellen in Anwesenheit von bovinen Gesamtmyelinproteinen;
- Auswählen und Expandieren von T-Zellen, die auf die Myelinproteine ansprechen; und
- Abschwächen der T-Zellen durch Bestrahlung.

## Revendications

1. Vaccin comprenant, en une quantité efficace pour supprimer la sclérose en plaques par administration à un être humain, des lymphocytes T atténués qui ciblent plus d'une protéine de myéline.

2. Vaccin selon la revendication 1, comprenant des lymphocytes T cultivés en présence de protéines de myéline bovine totale.

3. Vaccin selon la revendication 2, le vaccin étant préparé par sélection et développement de lymphocytes T qui réagissent à des protéines de myéline.

4. Vaccin selon la revendication 1, dans lequel les lymphocytes T sont dérivés de cellules mononucléées du sang périphérique autologues.

5. Vaccin selon la revendication 1, dans lequel les lymphocytes T sont atténués par irradiation.

6. Vaccin selon la revendication 4, dans lequel les lymphocytes T sont congelés avant l'atténuation.

7. Utilisation de lymphocytes T atténués qui ciblent plus d'une protéine de myéline pour la fabrication d'un médicament destiné à entraîner une réponse immunitaire chez un patient humain ayant une sclérose en plaques.

8. Utilisation selon la revendication 7, dans laquelle les lymphocytes T sont dérivés de cellules mononucléées du sang périphérique autologues.

9. Utilisation selon la revendication 7, dans laquelle les lymphocytes T comprennent des lymphocytes T cultivés en présence de protéines de myéline bovine totale.

10. Utilisation selon la revendication 9, dans laquelle les lymphocytes T sont préparés par sélection et développement de lymphocytes T qui réagissent à des protéines de myéline.

11. Utilisation selon la revendication 7, dans laquelle les lymphocytes T atténués sont atténués par irradiation.

12. Utilisation selon la revendication 7, dans laquelle les lymphocytes T sont administrés par voie sous-cutanée.

13. Utilisation selon la revendication 7, dans laquelle les lymphocytes T sont administrés à des intervalles de 4 à 6 semaines.

14. Utilisation selon la revendication 7, dans laquelle les lymphocytes T sont administrés pendant environ 18 mois.

15. Utilisation selon la revendication 7, dans laquelle les lymphocytes T sont administrés en une première dose de 30 × 10⁶ à 80 × 10⁶ lymphocytes T atténués.

16. Utilisation selon la revendication 15, comprenant en outre l'administration de plus d'une dose, les doses ultérieures étant augmentées s'il n'y a pas de réponse clinique à la première dose, jusqu'au moment de réactions défavorables ou d'une réponse clinique.

17. Vaccin comprenant des lymphocytes T atténués en une quantité efficace pour supprimer la sclérose en plaques par administration à un être humain, dans lequel les lymphocytes T atténués sont préparés par:
culture de cellules mononucléées du sang périphérique autologues en présence de protéines de myéline bovine totale;
sélection et développement des lymphocytes T qui réagissent aux protéines de myéline; et
atténuation des lymphocytes T par irradiation.
